# EUROPÄISCHE PATENTSCHRIFT

(11) **EP 0 765 859 B1**
(45) Veröffentlichungstag und Bekanntmachung des Hinweises auf die Patenterteilung: **13.12.2000**
(21) Anmeldenummer: 96115454.9
(22) Anmeldetag: 26.09.1996
(51) Int. Cl.: C07C 67/08, C07C 69/54

(54) **Verfahren und Vorrichtung zur kontinuierlichen Herstellung von Alkylestern der (Meth)acrylsäure**
Process and equipment for the continuous preparation of alkyl (meth-)acrylates
Procédé et installation de préparation continue de (méth-)acrylates d'alkyle

(30) Priorität: 28.09.1995 DE 19536178
(43) Veröffentlichungstag der Anmeldung: 02.04.1997
(73) Patentinhaber: BASF AKTIENGESELLSCHAFT, 67056 Ludwigshafen (DE)
(72) Erfinder: Exner, Herbert, Dr., 67165 Waldsee (DE); Baur, Karl Gerhard, Dr., 67063 Ludwigshafen (DE); Dockner, Toni, Dr., 67149 Meckenheim (DE); Potthoff, Christiane, 44141 Dortmund (DE); Dams, Albrecht, Dr., 67157 Wachenheim (DE)
(74) Vertreter: Isenbruck, Günter, Dr.

(56) Entgegenhaltungen:
- EP-A- 0 733 617
- DE-A- 2 252 334
- DE-A- 2 552 987
- DE-A- 3 308 879

## Beschreibung

Die Erfindung betrifft ein Verfahren zur kontinuierlichen Herstellung von Alkylestern der (Meth)acrylsäure durch Umsetzung von (Meth)acrylsäure und 1 bis 8 C-Atome aufweisenden einwertigen Alkanolen in homogener, flüssiger, an Lösungsmittel freier Phase bei erhöhter Temperatur und in Gegenwart eines sauren Veresterungskatalysators, bei dem man die (Meth)acrylsäure, das Alkanol und den Säurekatalysator kontinuierlich einer Reaktionszone zuführt, nach einer Verweilzeit den gebildeten (Meth)acrylsäurealkylester als Bestandteil wenigstens eines neben dem (Meth)acrylsäurealkylester als weiteren Bestandteilen aus Wasser oder Wasser und Ausgangsalkanol bestehenden Azeotrops über Kopf einer der Reaktionszone aufgesetzten Rektifikationszone rektifikativ abtrennt, das anfallende Destillat in wenigstens eine den (Meth)acrylsäurealkylester enthaltende organische und in wenigstens eine Wasser enthaltende wäßrige Phase auftrennt, einen Teil der (Meth)acrylsäurealkylester enthaltenden organischen Phase über Kopf der Rektifikationszone zurückführt und gegebenenfalls Wasser in die Reaktionszone zurückführt, aus der überschüssigen, den (Meth)acrylsäurealkylester enthaltenden organischen Phase den (Meth)acrylsäurealkylester abtrennt und einen Teil des Reaktionsgemisches aus der Reaktionszone kontinuierlich austrägt.

Der Begriff (Meth)acrylsäure bezeichnet in bekannter Weise Acryl- oder Methacrylsäure. Alkylester der (Meth)acrylsäure sind allgemein bekannt und z.B. als Ausgangsmonomere zur Herstellung wäßriger Polymerisatdispersionen von Bedeutung, die z.B. als Klebstoffe Verwendung finden.

Verfahren zur Herstellung von (Meth)acrylsäurealkylestern durch Umsetzung von (Meth)acrylsäure mit 1 bis 8 C-Atomen aufweisenden einwertigen Alkanolen in homogener flüssiger Phase, bei erhöhter Temperatur und in Gegenwart Protonen liefernder Katalysatoren sind bekannt und z.B. in den DE-A 14 68 932, 22 26 829 und 22 52 334 beschrieben. Es handelt sich hierbei um typische Gleichgewichtsreaktionen, bei denen der Umsetzungsgrad der (Meth)acrylsäure und des jeweiligen Alkanols zum entsprechenden Ester durch die Gleichgewichtskonstante signifikant begrenzt ist. Dies hat zur Folge, daß für eine wirtschaftliche Verfahrensführung die nicht umgesetzten Ausgangsstoffe vom gebildeten Ester abgetrennt und in die Reaktionszone zurückgeführt werden müssen. Als besonders schwierig erweist sich dabei in der Regel die Abtrennung des gebildeten Esters von nicht umgesetzter (Meth)acrylsäure, da deren Siedepunkte meist vergleichsweise eng zusammenliegen. Es wurden daher bereits verschiedene Maßnahmen zur Erhöhung des Umsatzes der (Meth)acrylsäure zu den entsprechenden Estern vorgeschlagen, wie z.B. die Anwendung eines erhöhten molaren Überschusses an Alkanol gegenüber der (Meth)acrylsäure, die Entfernung des Reaktionswassers mittels eines ein geeignetes Azeotrop bildenden organischen Schleppmittels oder die Extraktion des gebildeten Esters mit einem geeigneten Lösungsmittel während der Reaktion. Diese Verfahren weisen jedoch den Nachteil auf, daß ein großer Überschuß an Alkanol zurückgewonnen bzw. das Schleppmittel oder das Extraktionsmittel isoliert werden müssen. Ein erhöhter Alkanolüberschuß verstärkt zudem die Bildung von dessen Dialkylether als Nebenprodukt.

Inzwischen ist auch bekannt geworden, die Siedepunktsdifferenz zwischen nicht umgesetzter (Meth)acrylsäure und gebildetem Alkylester dadurch zu erhöhen, daß man den gebildeten Alkylester der (Meth)acrylsäure in wenigstens ein niedrig siedendes wäßriges Azeotrop einbettet, das neben dem (Meth)acrylsäurealkylester und Wasser noch Ausgangsalkanol enthalten kann, und den (Meth)acrylsäurealkylester als Bestandteil wenigstens eines solchen Azeotrops der, die nicht umgesetzte (Meth)acrylsäure umfassenden, Reaktionszone kontinuierlich rektifikativ zu entziehen, und so von nicht umgesetzter (Meth)acrylsäure abzutrennen. Bei kontinuierlicher Durchführung der vorstehenden Verfahrensweise wird das als Destillat anfallende wäßrige Azeotrop in eine wenigstens eine den (Meth)acrylsäurealkylester enthaltende organische und in wenigstens eine Wasser enthaltende wäßrige Phase aufgetrennt. Ein Teil der den (Meth)acrylsäurealkylester enthaltenden organischen Phase wird zur Einstellung der rektifikativen Trennwirkung (rektifikatives Rücklaufverhältnis) über Kopf der aufgesetzten Rektifikationszone zurückgeführt.

Handelt es sich bei dem im Rahmen des erfindungsgemäßen Verfahrens aus der Reaktionszone kontinuierlich über Kopf einer Rektifikationszone abgetrennten, den Zielester enthaltenden, wäßrigen Azeotrop um kein Heteroazeotrop, so scheidet sich dieses nach seiner Kondensation nicht von selbst in eine wäßrige und in eine organische Phase. Diese Auftrennung kann jedoch in einfacher Weise z.B. dadurch erzielt werden, daß man das im Azeotrop enthaltene Alkanol mittels Wasser extrahiert und das dabei anfallende Wasser/Alkanol-Gemisch rektifikativ auftrennt. Das Alkanol wird zweckmäßigerweise in die Reaktionszone zurückgeführt, vorzugsweise zur Einstellung der rektifikativen Trennwirkung über Kopf der aufgesetzten Rektifikationszone.

Aus der überschüssigen, den (Meth)acrylsäurealkylester enthaltenden, organischen Phase trennt man den (Meth)acrylsäurealkylester in an sich bekannter Weise ab. Bei der Veresterung niederer (C₁-, C₂-)Alkanole reicht normalerweise das im Verlauf der Veresterung als Nebenprodukt gebildete Reaktionswasser zur Ausbildung der Zusammensetzung des wäßrigen Azeotrops aus (geeignete wäßrige Azeotropzusammensetzungen weist z.B. Azeotropic Data-III, Advances in Chemistry, Series 116, American Chemical Society, Washington, D.C. (1973), aus) und wird so als Bestandteil des wäßrigen Azeotrops gleichzeitig kontinuierlich aus dem Veresterungsgleichgewicht entfernt. Mit zunehmender Kettenlänge des Alkanols ist dies jedoch nicht mehr der Fall, weshalb in diesen Fällen, über das im Verlauf der Veresterung gebildete Reaktionswasser hinaus, zusätzliches Wasser in die Reaktionszone eingeführt werden muß. In einfachster Weise wird dies dadurch realisiert, daß man einen entsprechenden Teil der bei Auftrennung des als Destillat anfallenden wäßrigen Azeotrops erzeugten wäßrigen Phase in den Veresterungsteil zurückführt. Da Wasser bezüglich des Umsatzes bei der eigentlichen Veresterungsreaktion eher stört, erfolgt die Rückführung der wäßrigen Phase vorzugsweise über Kopf der aufgesetzten Rektifikationszone. Enthält das über Kopf der aufgesetzten Rektifikationszone rektifikativ abgetrennte wäßrige Azeotrop als zusätzlichen Bestandteil Ausgangsalkanol, wird dieser aus dem nach Auftrennung des azeotropen Destillats in eine organische und eine wäßrige Phase nach deren Teilrückführung verbleibenden Überschuß an organischer und wäßriger Phase in an sich bekannter Weise abgetrennt und in die Reaktionszone zurückgeführt. Da das Ausgangsalkanol als einer der beiden Reaktanden unmittelbar an der Veresterung teilnimmt, erfolgt diese Rückführung mit Vorzug auf direktem Weg.

Die GB-1017522 offenbart ein entsprechendes Verfahren zur Herstellung von n-Butylacrylat. Als Veresterungsbedingungen empfiehlt die GB-1017522 dabei ein molares Verhältnis von Ausgangsalkanol zu Ausgangssäure von 2,3 bis 5, eine < 100°C betragende Reaktionstemperatur, sowie einen auf die Gesamtmasse der Reaktanden bezogenen Gehalt an katalytisch wirksamer Schwefel- oder organischer Sulfonsäure von > 0,05 bis < 5 Gew.-%. Nachteilig an dieser Verfahrensweise ist der erforderliche erhöhte Überschuß an Ausgangsalkanol, der die Bildung an unerwünschtem Dialkylether fördert, sowie die unter vorgenannten Bedingungen nicht voll befriedigende Ausbeute an n-Butylacrylat bezogen auf die eingesetzte Menge an Acrylsäure.

Aus der DE-PS 25 52 987 ist ein Verfahren zur kontinuierlichen Herstellung von Alkylestern der Acrylsäure durch Umsetzung von Acrylsäure und 1 bis 4 C-Atome aufweisenden einwertigen Alkanolen in homogener, flüssiger, an Lösungsmittel freier Phase im Molverhältnis von 1(Alkanol): 1(Acrylsäure) bis 2(Alkanol):1(Acrylsäure) bei erhöhter Temperatur und in Gegenwart von Schwefel- oder organischer Sulfonsäure als Katalysator bekannt, bei dem man die Acrylsäure, das Alkanol und den Säurekatalysator kontinuierlich einer Reaktionszone zuführt, nach einer Verweilzeit von einigen Stunden den gebildeten Acrylsäurealkylester als Bestandteil wenigstens eines neben dem Acrylsäurealkylester als weiteren Bestandteilen aus Wasser oder Wasser und Ausgangsalkanol bestehenden wäßrigen Azeotrops über Kopf einer der Reaktionszone aufgesetzten, einen Kopfdruck von 0,1 bis 1 atm aufweisenden Rektifikationskolonne rektifikativ abtrennt, das anfallende Destillat I in eine den gebildeten Acrylsäureester enthaltende organische und in eine wäßrige Phase auftrennt, einen Teil der organischen Phase zum Zwecke der Erzeugung einer erhöhten Trennwirkung sowie gegebenenfalls einen Teil der wäßrigen Phase zur Aufrechterhaltung der Zusammensetzung des wäßrigen Azeotrops über Kopf der Rektifikationszone zurückführt, aus der überschüssigen organischen Phase den Alkylester in an sich bekannter Weise abtrennt und einen Teil des Reaktionsgemisches aus der Reaktionszone austrägt, von Hochsiedern destillativ befreit und das dabei anfallende Destillat II in die Reaktionszone zurückführt.

Vorrangige Zielsetzung der DE-PS 25 52 987 ist die Vermeidung unerwünschter Etherbildung aus Ausgangsalkanol. Nachteilig an der Verfahrensweise der DE-PS 25 52 987 ist jedoch, daß trotz destillativer Behandlung des Austrags aus dem Reaktionsgemisch und Rückführung des dabei anfallenden Destillats in die Reaktionszone die Ausbeute an Alkylacrylat, bezogen auf eingesetzte Acrylsäure, nicht zu befriedigen vermag. Auch die erreichte Verringerung der Dialkylether-Nebenproduktbildung kann nicht voll befriedigen. Ferner vermag auch die in den Ausführungsbeispielen erforderliche Verweilzeit nicht zu befriedigen. Dies gilt auch für die Raum-Zeit-Ausbeute. Es wird angenommen, daß dies auf der geringen Konzentration an saurem Veresterungskatalysator beruht.

Es ist daher bereits vorgeschlagen worden (deutsche Patentanmeldung 195 10 891.4), das entsprechende Veresterungsverfahren im Beisein erhöhter Konzentrationen an saurem Veresterungskatalysator durchzuführen, was die Rückspaltung von bei der Veresterung als weitere Nebenprodukte gebildeten Oxiestern fördert und damit bei gegebener Verweilzeit die auf eingesetzte (Meth)acrylsäure bezogene Ausbeute an Ester erhöht.

Der Erfindung liegt die Aufgabe zugrunde, ein Veresterungsverfahren zur Herstellung von Alkylestern der (Meth)acrylsäure zu schaffen, das eine optimierte Ausbeute an Ester und gleichzeitig eine Minimierung der Dialkylethernebenproduktbildung ermöglicht.

Diese Aufgabe wird durch ein Verfahren zur kontinuierlichen Herstellung von Alkylestern der (Meth)acrylsäure durch Umsetzung von (Meth)acrylsäure und 1 bis 8 C-Atome aufweisenden einwertigen Alkanolen in homogener, flüssiger, an Lösungsmittel freier Phase bei erhöhter Temperatur und in Gegenwart eines sauren Veresterungskatalysators, bei dem man die (Meth)acrylsäure, das Alkanol und den Säurekatalysator kontinuierlich einer Reaktionszone zuführt, nach einer Verweilzeit den gebildeten (Meth)acrylsäurealkylester als Bestandteil wenigstens eines neben dem (Meth)acrylsäurealkylester als weiteren Bestandteilen aus Wasser oder Wasser und Ausgangsalkanol bestehenden Azeotrops über Kopf einer der Reaktionszone aufgesetzten Rektifikationszone rektifikativ abtrennt, das anfallende Destillat in wenigstens eine den (Meth)acrylsäurealkylester enthaltende organische und in wenigstens eine Wasser enthaltende wäßrige Phase auftrennt, einen Teil der (Meth)acrylsäurealkylester enthaltenden organischen Phase über Kopf der Rektifikationszone zurückführt und gegebenenfalls Wasser in die Reaktionszone zurückführt, aus der überschüssigen, den (Meth)acrylsäurealkylester enthaltenden organischen Phase den (Meth)acrylsäurealkylester abtrennt und einen Teil des Reaktionsgemisches aus der Reaktionszone kontinuierlich austrägt, gelöst, das dadurch gekennzeichnet ist, daß die Reaktionszone aus einer Kaskade von mindestens zwei hintereinander geschalteten, vorzugsweise kontinuierlich betriebenen, Reaktionsbereichen besteht, und der flüssige Austragstrom eines Reaktionsbereichs den Zulaufstrom des nachfolgenden Reaktionsbereichs bildet.

Bei diesem Verfahren nimmt aufgrund der relativen Schwerflüchtigkeit des sauren Veresterungskatalysators der auf die im jeweiligen Reaktionsbereich enthaltene Menge an Veresterungsgemisch bezogene Gewichtsanteil an saurem Veresterungskatalysator längs der Reaktionskaskade zu. Dies führt zu einer räumlichen Trennung von Veresterung und Rückspaltung und bringt eine reduzierte Dialkyletherbildung mit sich. Zum Erreichen dieses Säureprofils längs der Kaskade kann in die einzelnen Reaktionsbereiche aber auch zusätzlicher externer Säurezusatz erfolgen.

Eine Ausführungsform besteht darin, daß der flüssige Ausgangsstrom eines Reaktionsbereichs mit Hilfe einer Pumpe einem nachfolgenden Reaktionsbereich zugeleitet wird. Weitere Ausführungsformen bestehen darin, daß der Austrag auch mit Hilfe eines Überlaufs in einen nachfolgenden Reaktionsbereich erfolgt oder auch in den unteren Teil der Rektifikationskolonne geleitet wird, von wo die von Leichtsiedern befreite Rektifikationsmischung in den Reaktionsbereich fließt, der mit der Rektifikationskolonne eine Flüssigkeitsverbindung besitzt.

Gemäß einer vorteilhaften Ausbildung der Erfindung beträgt der Gehalt an katalytisch wirksamer Säure im ersten Reaktionsbereich, bezogen auf das darin enthaltene Reaktionsgemisch, 0,1 bis 5 Gew.-% Schwefelsäure (bzw. eine dazu äquimolare Menge an organischer Sulfonsäure, vorzugsweise Methansulfonsäure, Benzolsulfonsäure, Dodecylbenzolsulfonsäure und/oder p-Toluolsulfonsäure oder an einem Gemisch aus organischer Sulfonsäure und Schwefelsäure). Der entsprechende Säuregehalt im letzten Reaktionsbereich beträgt vorzugsweise > 5 bis 20 Gew.-%. Die Gesamtverweilzeit der Reaktanden in der Kaskade beträgt in der Regel 1 bis 20 h.

Für den Fall, daß es sich bei den einzelnen Reaktionsbereichen um räumlich voneinander getrennte Reaktoren handelt, ist deren Anzahl unter Berücksichtigung der Investitionskosten vorteilhafterweise ≥ 2 und ≤ 4. Wird mehr als ein Reaktionsbereich innerhalb ein und desselben Reaktors geschaffen (z.B. durch Einsatz von Trennblechen), so kann die Anzahl von Reaktionsbereichen auch größer als 4 sein.

Eine mögliche Ausgestaltung des erfindungsgemäßen Verfahrens besteht darin, daß der gesamten Reaktionszone nur eine Rektifikationskolonne aufgesetzt wird, deren flüssiger Rücklauf lediglich mit dem ersten Reaktionsbereich verbunden ist. Bezüglich der übrigen Reaktionsbereiche besteht lediglich für die aufsteigenden Dämpfe eine Verbindung zur Rektifikationskolonne. Eine weitere Ausgestaltungsform besteht darin, daß der erste Reaktionsbereich als Verweilzeitbehälter (Vorreaktor) ohne Verbindung zur Rektifikationskolonne betrieben wird. Vorzugsweise ist der erste Reaktionsbereich gasseitig weder mit einem nachfolgenden Reaktionsbereich noch mit der Rektifikationszone verbunden. Der Kopfdruck der Rektifikationskolonne beträgt zweckmäßigerweise 0,1 bis 1 atm, wobei Atmosphärendruck besonders bevorzugt ist.

Die Temperatur des Reaktionsgemisches in den verschiedenen Reaktionsbereichen entspricht normalerweise der dem eingestellten Druck entsprechenden Siedetemperatur des jeweiligen Reaktionsgemisches. D.h. sie nimmt normalerweise längs der Kaskade zu, was hinsichtlich der Verfahrensziele von Vorteil ist. Im ersten Reaktionsbereich liegt die Temperatur normalerweise bei ≥ 80°C und ≤ 125°C. Im letzten Reaktionsbereich der Kaskade sollte sie ≤ 135°C betragen, um unerwünschte Polymerisation als Nebenreaktion zu unterdrücken.

Die auf die Menge der eingesetzten (Meth)acrylsäure bezogenen Umsätze liegen typischerweise bei ≥ 95 mol.-%. Mit Vorteil wird im ersten Reaktionsbereich bereits bei einem ≥ 90 mol.-% liegenden Umsatz gefahren. Bei Verwendung eines Verweilzeitreaktors (Vorreaktor) als erstem Reaktionsbereich siedet das Reaktionsgemisch in diesem in der Regel nicht. Als Reaktionstemperatur ist auch hier die Temperatur von 80°C bis 125°C zu empfehlen. Bei Verwendung eines Verweilzeitreaktors als erstem Reaktionsbereich wird in der Regel ein Umsatz von ca 80% des Gleichgewichtswerts erreicht. Vorteilhafterweise sind die Reaktionsbereiche so zu gestalten, daß die Verweilzeit längs der Kaskade von Reaktionsbereich zu Reaktionsbereich abnimmt.

Gemäß einer vorteilhaften Ausbildung der Erfindung beträgt das molare Einsatzverhältnis von Alkanol zu (Meth)acrylsäure ≥ 1 und liegt in der Regel unterhalb von 5. Besonders vorteilhaft ist ein Verhältnis von 1:1 bis 3:1. Von ganz besonderem Vorteil ist ein Verhältnis von 1,1:1 bis 1,8:1. Besonders vorteilhaft ist es, als Veresterungskatalysatoren Schwefelsäure und/oder organische Sulfonsäuren einzusetzen.

Um den Anteil an hochsiedenden nicht spaltbaren Nebenprodukten zu begrenzen genügt es in der Regel ≤ 2,5 Gew.-%, bezogen auf die gewonnene Estermenge, an Reaktionsgemisch aus der Reaktionszone kontinuierlich auszuschleusen, bevorzugt aus dem letzten Reaktionsbereich.

Mit Vorteil wird aus dem letzten Reaktionsbereich der Kaskade ein Teilstrom in den ersten und je nach Bedarf in andere Reaktionsbereiche rückgeführt. Mit der Ausschleusung der Hochsieder geht saurer Veresterungskatalysator mit, der kontinuierlich in das Reaktionsgemisch ergänzt wird. Dabei geht auch Prozeßstabilisator mit, so daß sich dessen Gehalt auf einen stationären Wert einpendelt. Dies führt zu stationären Zuständen der erforderlichen Konzentration an saurem Veresterungskatalysator. Gleichzeitig wird durch diese Kreisführung eine Katalysatoraufbereitung überflüssig und der Bedarf an frischem Katalysator reduziert.

Da die eingesetzte Acrylsäure geringe Anteile an Essigsäure enthält, fallen neben Dialkylether als Nebenprodukte Alkylester der Essigsäure an. Beide Nebenkomponenten gehen über Kopf der aufgesetzten Rektifikationskolonne mit und verbleiben bei der destillativen Alkanol/Ester Auftrennung im Alkanol, das zweckmäßigerweise ins Ausgangsgemisch rückgeführt wird. Dadurch reichern sich beide Verunreinigungen in den Rückströmen an. Stationäre Konzentrationen stellen sich dadurch ein, daß in gewissem Umfang diese Leichtsieder sich in den resultierenden Ester drücken. Je nach Reinheitsanforderungen an den Ester wird daher auch aus dem Alkanolkreis eine Ausschleusung vorgenommen.

In einer der Alkanol/Ester-Auftrennung nachgeschalteten einfachen Destillationskolonne kann durch destillatives Abtrennen des Esters dieser von den Prozeßstabilisatoren getrennt und auf Lagerstabilisatoren umgerüstet werden.

Vorzugsweise ist die zu veresternde Säure Acrylsäure und/oder das zu veresternde Alkanol ein C₁₋₄-Alkanol, insbesondere n-Butanol.

Besonders bevorzugt wird das erfindungsgemäße Verfahren zur Herstellung von n-Butylacrylat angewendet.

Die in der erfindungsgemäß als Kaskade aufgebauten Reaktionszone entstehenden Dämpfe werden, wie bereits beschrieben, einer Rektifikationszone kontinuierlich zugeführt. Hinsichtlich des aus dieser über Kopf abgetrennten, den Zielester enthaltenden, wäßrigen Azeotrops lassen sich im wesentlichen zwei Fallgestaltungen unterscheiden. Handelt es sich um ein Heteroazeotrop, wie z.B. im Fall der Herstellung von n-Butylacrylat, scheidet sich das Azeotrop nach seiner Kondensation von selbst in eine wäßrige und in eine organische Phase. Die wäßrige Phase besteht normalerweise hauptsächlich aus Wasser und etwas Alkanol, die organische Phase besteht in der Regel im wesentlichen aus dem gebildeten Ester und Alkanol. Zur Einstellung der rektifikativen Trennwirkung führt man einen entsprechenden Teil der organischen Phase über Kopf der Rektifikationszone zurück.

Zur Aufrechterhaltung der Zusammensetzung des wäßrigen Azeotrops führt man einen entsprechenden Teil der wäßrigen Phase in die Reaktionszone zurück, vorzugsweise ebenfalls über Kopf der aufgesetzten Rektifikationszone. Aus dem nicht rückgeführten Anteil der wäßrigen Phase läßt sich enthaltenes Alkanol z.B. durch Strippen (z.B. mit Luft) abtrennen und in die Reaktionszone zurückführen. Zweckmäßigerweise erfolgt die Rückführung auf direktem Weg. Das dabei anfallende im wesentlichen reine Wasser wird ausgetragen. Aus dem nicht rückgeführten Teil der organischen Phase wird der gebildete (Meth)acrylsäurealkylester in an sich bekannter Weise, z.B. gemäß der DE-A 25 52 987, abgetrennt. D.h. beispielsweise führt man die überschüssige organische Phase einer nachgeschalteten Rektifikationskolonne zu und trennt das Alkanol über Kopf in reiner Form oder als aus Ester und Alkanol bestehendes Azeotrop ab. Vorzugsweise führt man das so abgetrennte Alkanol (in reiner oder in azeotroper Form) in die Reaktionszone zurück. Zweckmäßigerweise erfolgt die Rückführung auf direktem Weg.

Die Sumpfflüssigkeit dieser Rektifikationskolonne besteht im wesentlichen aus dem gewünschten Ester sowie geringen Mengen an tiefer und höher als dieser Ester siedenden Nebenprodukten. Man bezeichnet die Sumpfflüssigkeit dieser Rektifikationskolonne deshalb auch als Roh-(Meth)acrylsäurealkylester. Bei den tiefer siedenden Nebenprodukten handelt es sich insbesondere um den Dialkylether und den Essigsäure-ester des Ausgangsalkanols, da die Veresterung von (Meth)acrylsäure mit Alkanolen üblicherweise ausgehend von Roh-(Meth)acrylsäure erfolgt. Diese wird überwiegend durch katalytische Gasphasenoxidation von C₃-/C₄-Ausgangsverbindungen wie Propen oder iso-Buten erzeugt, wobei als Nebenprodukt in untergeordneten Mengen Essigsäure entsteht (vgl. z.b. DE-A 44 36 243). Höher siedende Nebenprodukte sind z.B. Oligo- und Polymerisate des α,β-monoethylenisch ungesättigten Zielesters.

In einer nachgeschalteten Leichtsieder-Rektifikationskolonne trennt man von dem Roh-(Meth)acrylsäurealkylester üblicherweise über Kopf die leichter siedenden Nebenprodukte ab, bevor in einer dieser nachgeschalteten Schwersieder-Rektifikationskolonne der gewünschte Rein-(Meth)acrylsäurealkylester über Kopf abgetrennt werden kann. Die die schwerer siedenden Nebenprodukte enthaltende Sumpfflüssigkeit der Schwersieder-Rektifikationskolonne wird zweckmäßigerweise in die Reaktionszone zurückgeführt, vorzugsweise auf direktem Weg.

Eine besondere Ausführungsform besteht darin, daß im Falle eine Acetatabtrennung aus dem Rückbutanolstrom, der wie vorher beschrieben erfolgt, der Ester aus der Rektifikationskolonne zur Rückgewinnung von Butanol durch einen Seitenabzug nach Abscheidung eventuell mitgerissener Flüssigkeitströpfchen entnommen und zum Reinester kondensiert werden. Bei der Kondensation wird diesem der Lagerstabilisator zugegeben (z.B. Hydrochinonmonomethylether). In diesem Ausführungsfall wird der Sumpfaustrag der Rektifikationskolonne zur Butanolrückgewinnung (in der Hauptsache bestehend aus Alkylacrylat) zweckmäßigerweise in den letzten Reaktionsbereich der Kaskade zur Spaltung von geringfügig enthaltenen Hochsiedern sowie zur Rückgewinnung von Reinester zurückgeführt.

In beiden Fällen wird zur Aufrechterhaltung eines stationären Zustands aus diesem letzten Reaktionsbereich der Kaskade, vorzugsweise kontinuierlich, eine geringe Menge an Reaktionsgemisch ausgetragen, um eine dortige Anreicherung an Schwersiedern zu vermeiden. Die dabei mit ausgetragene Menge an Säurekatalysator wird in die Reaktionszone ergänzt.

Üblicherweise enthält das aus der Reaktionszone abgezogene wäßrige Azeotrop, bei richtiger Einstellung der rektifikativen Trennwirkung, keine Ausgangssäure. Sollte letzteres jedoch nicht der Fall sein, läßt sich diese zusammen mit dem Alkanol extraktiv mittels Wasser abtrennen und das Extrakt anschließend in an sich bekannter Weise rektifikativ auftrennen. Beim erfindungsgemäßen Verfahren erfolgen sowohl die Veresterungsreaktion als auch die rektifikativen Trennungen und Extraktionen vorzugsweise in Gegenwart von üblichen Mengen an sich üblicher Polymerisationsinhibitoren. In der Regel werden, bezogen auf die Menge der α,β-monoethylenisch ungesättigten Monomeren, 0,01 bis 0,1 Gew.-% eines geeigneten Polymerisationsinhibitors eingesetzt. Sie werden mit Vorteil am Kopf der aufgesetzten Rektifikationskolonne und am Kopf der Alkanol/Ester Trennkolonne zugegeben. Als Polymerisationsinhibitoren kommen z.B. in Betracht phenolische Verbindungen wie Hydrochinon, Hydrochinonmonomethylether, aber auch p-Benzochinon, Phenothiazin, Methylenblau, Phenylendiamine und/oder Luft.

Das erfindungsgemäße Verfahren zeichnet sich im Vergleich zu den Verfahren des Stands der Technik durch eine deutliche Reduzierung von Teilschritten und Trennoperationen, durch reduzierte Verweilzeiten, eine erhöhte Ausbeute an gewünschtem Ester bezogen auf eingesetzte Ausgangssäure, eine reduzierte Ethermenge, einen reduzierten Austrag der Flüssigphase aus der Reaktionszone, sowie dadurch aus, daß es nicht mehr in notwendiger Weise einer Aufarbeitung dieses Austrags aus der Reaktionszone bedarf. Sowohl letzteres als auch die erhöhte Ausbeute sind wohl darauf zurückzuführen, daß der sich in der Reaktionszone einstellende stationäre Zustand kinetisch kontrolliert ist. Dabei spielt wahrscheinlich die Rückspaltung von relativ hochsiedenden Oxiestern (z.B. Alkoxypropionsäureester oder Acyloxipropionsäureester) eine zentrale Rolle.

Eine günstige Ausführungsform besteht darin, daß das Gesamtverfahren mit insgesamt vier Trennkolonnen zur Entfernung von Hochsiedern und Leichtsiedern betrieben wird. Diese sind: die Kolonne zur Entfernung des Esters über Kopf, die Butanol-Rückgewinnungskolonne, die Acetatkolonne zur Entfernung von Essigestern aus dem Rückbutanolstrom sowie eine Strippkolonne zur Entfernung von Restbutanol aus dem ausgeschleusten Reaktionswasser.

Weitere Einzelheiten und Vorteile der Erfindung können den anhand der Zeichnung beschriebenen Ausführungsbeispielen entnommen werden. Es sei an dieser Stelle nochmals betont, daß alle vorstehenden und nachfolgenden Ausführungen, insbesondere Gültigkeit für das Verfahren zur Herstellung von n-Butylacrylat besitzen, d.h. Butanol steht für n-Butanol.

### Beispiel 1

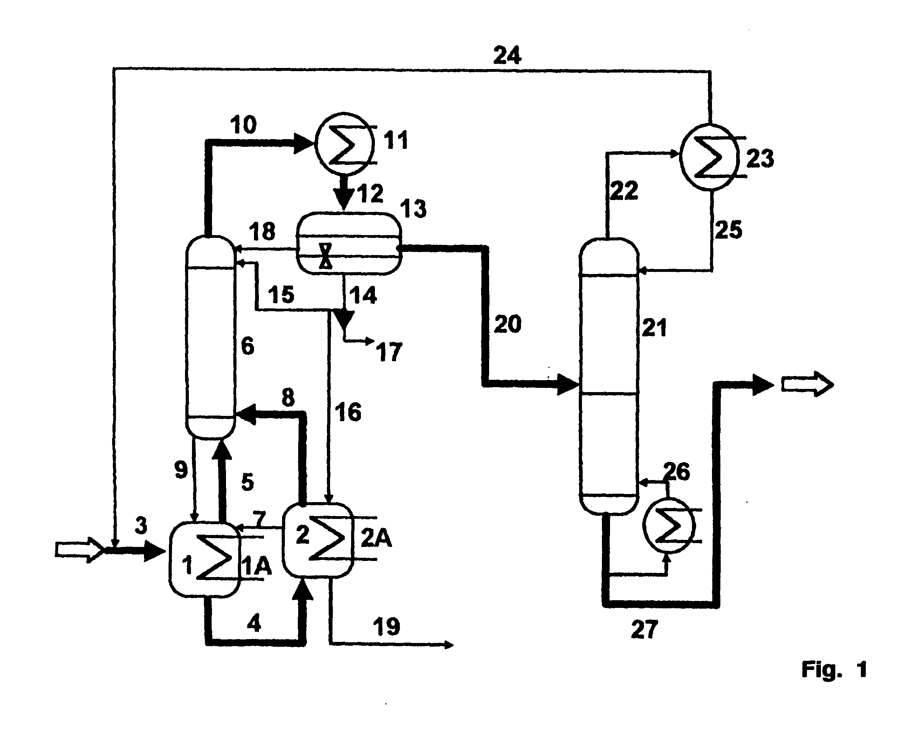

Dieses Ausführungsbeispiel soll im folgenden anhand der Figur 1 erläutert werden. Hier wurde einem Reaktor 1 mit Zwangsumlaufverdampfer 1A über eine Zuführungsleitung 3 ein Gemisch aus 5 mol/h Acrylsäure (AS), und 7 mol/h Butanol (BuOH) sowie Schwefelsäure als Veresterungskatalysator zugeführt. Die Menge an Schwefelsäure betrug 2 bis 3 Gew.-% bezogen auf das Reaktionsgemisch. Die Umsetzung wurde bei einer Temperatur von 120°C, einem Druck von 1 atm und einer Verweilzeit von 2,5 h durchgeführt. Das gasförmige Reaktionsprodukt wurde vom Kopf des Reaktors 1 über eine Leitung 5 einer Rektifikationskolonne zugeführt. Vom unteren Teil des Reaktors 1 wurde mittels einer (nicht dargestellten) Umwälzpumpe kontinuierlich ein Teilstrom des Reaktionsgemisches (Strom 4) in den unteren Teil eines zweiten Reaktors 2 mit Zwangsumlaufverdampfer 2A gepumpt. Dieser zweite Reaktor wurde bei der höheren Temperatur von 130°C sowie einer höheren Schwefelsäurekonzentration von ca. 10 Gew.-% betrieben. Der Reaktor 2 besaß einen Überlauf 7 zu Reaktor 1, durch den im Reaktor 1 eine konstante Katalysatorkonzentration aufrechterhalten wurde. Der Reaktor 2 besaß ebenfalls eine gasseitige Verbindung 8 zum unteren Teil der Destillationskolonne 6. Deren flüssiger Ablauf wurde über eine Leitung 9 in den Reaktor 1 geleitet. Im Reaktor 2 erfolgte die Nachreaktion und die In-situ-Spaltung gebildeter Oxiverbindungen (Michael-Addukte). Die beiden durch die Leitung 4 an ihrem unteren Ende miteinander verbundenen Reaktoren 1 und 2 bildeten die erfindungsgemäße, als Kaskade aufgebaute Reaktionszone. Das aus dieser Reaktionszone kontinuierlich in die Destillationskolonne 6 strömende Gas wurde dort rektifiziert, und das vom Kopf dieser Destillationskolonne 6 über die Leitung 10 abströmende, den zu bildenden Zielester enthaltende wäßrige Azeotrop, das aus Butylacrylat, Butanol und Wasser bestand, wurde einem Kondensator 11 zugeführt und darin kondensiert. Das Kondensat strömte über eine Leitung 12 in einen Abscheider 13. Dort schied sich das Azeotrop in eine wäßrige und in eine organische Phase. Die wäßrige Phase, die hauptsächlich aus Wasser und etwas Butanol bestand, wurde durch die Leitung 14 abgezogen und teilweise über die Leitung 15 dem Kopf der Destillationskolonne 6 zugeführt, um die rektifikatorische Trennung wie auch die Azeotropbildung der dort aufsteigenden Dämpfe zu bewirken. Ein anderer Teil der wäßrigen Phase wurde über die Leitung 16 dem Kopf des Reaktors 2 zugeführt. Ein weiterer Teil wurde über Leitung 17 zur Abstrippung von Butanol entnommen. Bei der Versuchsanordnung wurde die durch die Leitung 14 aus dem Kondensator abströmende Rücklaufmenge je zur Hälfte über die Leitung 15 in die Destillationskolonne und über die Leitung 16 in den Reaktor 2 geführt. Aus dem Abscheider 13 wurde organischer Rücklauf über die Leitung 18 in den Kopf der Destillationskolonne 6 eingeführt, um die Acrylsäure zurückzudrängen. Der wäßrige Rücklauf erfolgte temperaturgeregelt, der organische Rücklauf standgeregelt. Die am unteren Teil des Reaktors 2 vorgesehene Leitung 19 diente der Entsorgung verbliebener Rückstände.

Die im wesentlichen aus Butanol und Butylacrylat bestehende organische Phase enthielt nur 876 ppm (ppm Angaben beziehen sich stets auf das Gewicht) Dibutylether. Sie wurde kontinuierlich über eine Leitung 20 aus dem Abscheider 13 abgezogen und einer weiteren Kolonne 21, die in der Versuchsanordnung als Glockenbodenkolonne ausgebildet war, als Zulauf zugeführt, um Butanol zurückzugewinnen. In dieser Butanol-Rückgewinnungskolonne 21 wurde das überschüssige Butanol mit Resten von Wasser sowie Teilen von Butylacrylat durch die Leitung 22 über Kopf abgezogen und in einen Kondensator 23 geführt. Aus diesem wurde über Leitung 24 das überschüssige Butanol dem durch die Leitung 3 in den Reaktor 1 eingeführten Gemisch der Reaktanden beigefügt. Ein Teil des Destillats wurde aus dem Abscheider 23 über die Leitung 25 dem Kopf der Kolonne 21 als Rücklauf zur Erhöhung der rektifikativen Trennung zugeführt.

Aus dem Sumpf der Butanol-Rückgewinnungskolonne 21 wurde über eine Leitung 27 Roh-Butylacrylat entnommen, das frei von Hochsiedern war und nur Reste von Leichtsiedern aufwies und einen Gehalt an Butylacrylat von 99,5 Gew.-% aufwies. Nach einer anschließenden Leichtsieder- und Hochsiederabtrennung konnte ein reines Butylacrylat mit einer Reinheit von > 99,9 Gew.-% gewonnen werden. Die Butanol-Rückgewinnungskolonne 21 wurde bei 400 mbar betrieben. Der Reinester enthielt nur noch 598 ppm Dibutylether. Im Sumpf dieser Kolonne wurde mittels des Wärmetauschers 26 eine Temperatur von 115°C eingestellt.

Ein zweiter Versuch unter gleichen Bedingungen mit lediglich abgesenktem Butanolüberschuß (molar 1,3:1) ergab eine Absenkung des Ethers im Strom 20 auf 623 ppm bzw. eine Abreicherung im Reinprodukt von 374 ppm. Die Ausbeute an Butylacrylat bezogen auf Acrylsäure lagen beim ersten Versuch bei 98,8 %, d. Th. (der Theorie), beim zweiten Versuch bei 97,3 %, d. Th.

### Vergleichsbeispiel

Zum Vergleich wurde ein bekanntes Veresterungsverfahren mit nur einem Reaktionsbereich durchgeführt. Bei diesem Vergleichsbeispiel wurden in einem Umlaufverdampfer (2,5 l, Füllstand 2 1) stündlich 4 mol/h Acrylsäure, 4 mol/h Frisch-Butanol und 1,6 mol/h Rück-Butanol (Destillat der Butanolrückgewinnung) eingespeist. Die Verweilzeit betrug 2,5 h. Die Veresterung erfolgte im Beisein von 9,7 Gew.-% (bezogen auf das Reaktionsgemisch) Schwefelsäure bei einer Temperatur von 126°C. Der Reaktor war verbunden mit einer Glockenbodenkolonne (30 Böden), die mit 15 ml/h Hydrochinonmonomethylether (MeHQ) in Butanol stabilisiert wurde. Gebildetes Butylacrylat wurde über Kopf abgezogen, indem 340 ml/h Wasser als Rücklauf (Azeotropschleppmittel) auf die Kolonne gegeben wurde. Zusätzlich wurde 175 ml/h organischer Rücklauf auf die Kolonne gegeben, um die Acrylsäure zurückzudrängen.

Die überschüssige organische Phase (610 ml/h) wurde wie im Beispiel zum erfindungsgemäßen Verfahren zur Rückgewinnung von Butanol (BuOH) kontinuierlich in eine zweite Glockenbodenkolonne als Zulauf gegeben. Im Sumpf der Butanolkolonne dessen Temperatur 115°C betrug, wurde ein Roh-Ester mit einem Gehalt an Dibutylether von 1295 ppm erhalten. Im Zulauf zur BuOH-Kolonne waren 1940 ppm Ether. Die Kolonne wurde mit MeHQ (2 Gew.-% in Butanol) stabilisiert.

Nach der Leichtsieder- und Hochsiederabtrennung wurde ein Reinester mit einer Reinheit von > 99,9 Gew.-% gewonnen. Die Ausbeute bezogen auf Acrylsäure betrug 98,4 % d.Th.

### Beispiel 2

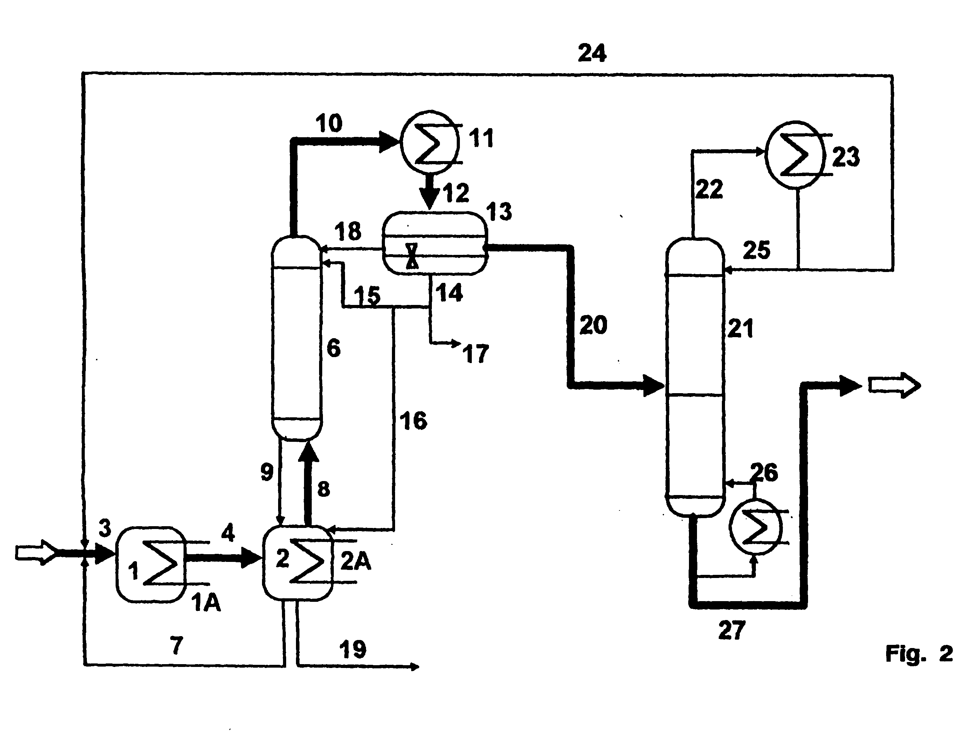

Dieses Ausführungsbeispiel soll anhand der Fig. 2 erläutert werden. Hier wurden für gleiche oder entsprechende Teile gleiche Bezugszeichen wie in Fig. 1 verwendet.

In einem Reaktor (Verweilzeitreaktor) 1 mit Zwangsumlaufverdampfer 1A wurde über eine Zuführleitung 3 ein Gemisch aus 5 mol/h Acrylsäure (AS), und 5,5 mol/h n-Butanol (BuOH) sowie Schwefelsäure als Veresterungskatalysator zugeführt. Das Verhältnis BuOH / AS betrug 1,1:1 (molar). Die Menge an Schwefelsäure betrug 2 bis 3 Gew.-% (bezogen auf das Reaktionsgemisch). Die Umsetzung im Reaktor 1 wurde bei einer Temperatur von 120°C und einer Verweilzeit von 3,5 h durchgeführt. Vom Reaktor 1 wurde kontinuierlich ein Teilstrom der flüssigen Reaktionsmischung über die Leitung 4 in den zweiten Reaktor 2 geleitet. Dieser zweite Reaktor wurde bei der höheren Temperatur von 130°C sowie bei einer höheren Schwefelsäurekonzentration von ca. 10 Gew.-% betrieben. Der Reaktor 2 besaß eine Flüssigkeitsrückführung 7 zum Reaktor 1, durch die im Reaktor 1 eine konstante Katalysatorkonzentration aufrechterhalten wurde. Der Reaktor 2 besaß eine gasseitige Verbindung 8 zum unteren Teil der Destillationskolonne 6. Deren flüssiger Ablauf wurde über eine Leitung 9 in den Reaktor 2 geleitet. Im Reaktor 2 erfolgte die Nachreaktion und die In-situ-Spaltung gebildeter Oxiverbindungen (Michael-Addukte). Die beiden durch die Leitung 4 miteinander verbundenen Reaktoren 1 und 2 bildeten die erfindungsgemäße, als Kaskade aufgebaute Reaktionszone.

Die Aufarbeitung des Reaktionsgemisch erfolgte analog der im ersten Beispiel beschriebenen Isolierung von Reinester. Der der Butanolrückgewinnungskolonne 21 am unteren Ende entnommene Rohester enthielt vor der Leichtsieder- und Hochsiederabtrennung einen Gehalt von 134 ppm Dibutylether. Der Reinester besaß einen Gehalt von 99,9 Gew.-% Butylacrylat. Die Ausbeute an Butylacrylat betrug 98,1 % d. Th. (bezogen auf Acrylsäure).

## Patentansprüche

1. Verfahren zur kontinuierlichen Herstellung von Alkylestern der (Meth)acrylsäure durch Umsetzung von (Meth)acrylsäure und 1 bis 8 C-Atome aufweisenden einwertigen Alkanolen in homogener, flüssiger, an Lösungsmittel freier Phase bei erhöhter Temperatur und in Gegenwart eines sauren Veresterungskatalysators, bei dem man die (Meth)acrylsäure, das Alkanol und den Säurekatalysator kontinuierlich einer Reaktionszone zuführt, nach einer Verweilzeit den gebildeten (Meth)acrylsäurealkylester als Bestandteil wenigstens eines neben dem (Meth)acrylsäurealkylester als weiteren Bestandteilen aus Wasser oder Wasser und Ausgangsalkanol bestehenden Azeotrops über Kopf einer der Reaktionszone aufgesetzten Rektifikationszone rektifikativ abtrennt, das anfallende Destillat in wenigstens eine den (Meth)acrylsäurealkylester enthaltende organische und in wenigstens eine Wasser enthaltende wäßrige Phase auftrennt, einen Teil der (Meth)acrylsäurealkylester enthaltenden organischen Phase über Kopf der Rektifikationszone zurückführt und Wasser in die Reaktionszone zurückführt, aus der überschüssigen, den (Meth)acrylsäurealkylester enthaltenden organischen Phase den (Meth)acrylsäurealkylester abtrennt und einen Teil des Reaktionsgemisches aus der Reaktionszone kontinuierlich austrägt, dadurch gekennzeichnet, daß die Reaktionszone aus einer Kaskade von mindestens zwei hintereinander geschalteten Reaktionsbereichen besteht, und der Austragstrom eines Reaktionsbereichs einen Zulaufstrom eines nachfolgenden Reaktionsbereichs bildet.

2. Verfahren nach Anspruch 1, dadurch gekennzeichnet, daß die Kaskade zwei bis vier voneinander räumlich getrennte Reaktionsbereiche aufweist und/oder daß die aufsteigenden Dämpfe aus den Reaktionsbereichen einer einzelnen Rektifikationszone zugeführt werden, deren flüssiger Rücklauf nur in den ersten Reaktionsbereich zurückgeführt wird, und/oder daß der erste Reaktionsbereich gasseitig weder mit einem nachfolgenden Reaktionsbereich noch mit der Rektifikationszone verbunden ist.

3. Verfahren nach Anspruch 1 oder 2, dadurch gekennzeichnet, daß die Temperatur im ersten Reaktionsbereich zwischen 80°C und 125°C beträgt und im letzten Reaktionsbereich unter 135°C liegt, und/oder daß der Kopfdruck der Rektifikationskolonne zwischen 0,1 und 1 atm, vorzugsweise bei 1 atm, liegt, und/oder daß die Gesamtverweilzeit der Reaktanden in der Kaskade zwischen 1 und 20 Stunden beträgt.

4. Verfahren nach einem der vorhergehenden Ansprüche, dadurch gekennzeichnet, daß das molare Einsatzverhältnis von Alkanol zu (Meth)acrylsäure ≥ 1, vorzugsweise 1:1 bis 3:1, vorzugsweise 1,1:1 bis 1,8.1, beträgt.

5. Verfahren nach einem der vorhergehenden Ansprüche, dadurch gekennzeichnet, daß als Veresterungskatalysatoren Schwefelsäure und/ oder organische Sulfonsäuren, vorzugsweise Methansulfonsäure, Benzolsulfonsäure, Dodecylbenzolsulfonsäure und/oder p-Toluolsulfonsäure eingesetzt werden.

6. Verfahren nach einem der vorhergehenden Ansprüche, dadurch gekennzeichnet, daß der Gehalt an katalytisch wirksamer Säure im ersten Reaktorbereich bezogen auf das darin enthaltene Reaktionsgemisch 0,1 bis 5 Gew.-% Schwefelsäure und/oder einer äquimolaren Menge an organischer Sulfonsäure beträgt, und/oder daß der Gehalt an katalytisch wirksamer Säure im letzten Reaktionsbereich bezogen auf das darin enthaltene Reaktionsgemisch zwischen 5 und 20 Gew.-% Schwefelsäure und/oder einer äquimolaren Menge an organischer Sulfonsäure beträgt, und/oder daß die zu veresternde Säure Acrylsäure ist, und/oder daß das zu veresternde Alkanol ein C₁- bis C₄-Alkanol ist, vorzugsweise n-Butanol ist.

7. Vorrichtung zur Durchführung des Verfahrens zur kontinuierlichen Herstellung von Alkylestern der (Meth)acrylsäure nach einem der Ansprüche 1 bis 6 mit einem ersten Reaktor (1), der mit einer Zuführungsleitung (3) für die Reaktanden versehen ist und dessen Kopf über eine Leitung (5) mit dem unteren Teil einer Rektifikationskolonne (6) verbunden ist, deren Kopf über einen Kondensator mit einem Abscheider (13) in Verbindung steht, der eine Abführleitung (20) für das Rohprodukt aufweist, dadurch gekennzeichnet, daß das untere Ende des ersten Reaktors (1) über eine Leitung (4) mit dem unteren Ende mindestens eines weiteren Reaktors (2) verbunden ist, dessen Kopf über eine Leitung (8) mit dem unteren Teil der Rektifikationssäule (6) in Verbindung steht, und daß der obere Teil des Abscheiders (13) mit dem Kopfteil der Rektifikationskolonne (6) über eine Leitung (18) und der untere Teil des Abscheiders (13) mit dem Kopfteil der Rektifikationskolonne (6) über eine Leitung (14/15) und/oder dem Kopfteil mindestens eines des weiteren Reaktoren (2) über eine Leitung (14/16) verbunden ist.

8. Vorrichtung nach Anspruch 7, dadurch gekennzeichnet, daß der obere Teil mindestens eines weiteren Reaktors (2) über eine Leitung (7) mit dem oberen Teil des ersten Reaktors (1) verbunden ist, und/oder daß der untere Teil der Rektifikationskolonne (6) über eine Leitung (9) mit dem Kopfteil des ersten Reaktors (1) verbunden ist, und/oder daß der Abscheider (13) mit einer weiteren Rektifikationskolonne (21) verbunden ist.

9. Vorrichtung zur Durchführung des Verfahrens zur kontinuierlichen Herstellung von Alkylestern der (Meth)acrylsäure nach einem der Ansprüche 1 bis 6 mit einem Reaktor (1), der mit einer Zuführungsleitung (3) für die Reaktanden versehen ist und der als Verweilzeitbehälter den ersten Reaktionsbereich bildet, dadurch gekennzeichnet, daß der erste Reaktor (1) eine flüssigseitige Verbindung (4) mit mindestens einem weiteren Reaktor (2) besitzt, dessen Kopf über eine Leitung (8) mit dem unteren Teil der Rektifikationskolonne (6) in Verbindung steht, deren Kopf über einen Kondensator mit einem Abscheider (13) in Verbindung steht, der eine Abführleitung (20) für das Rohprodukt aufweist, und daß des obere Teil des Abscheiders (13) mit dem Kopfteil der Rektifikationskolonne (6) über eine Leitung (18) und der untere Teil des Abscheiders (13) mit dem Kopfteil der Rektifikationskolonne (6) über eine Leitung (14/15) und/oder dem Kopfteil mindestens eines des weiteren Reaktoren (2) über eine Leitung (14/16) verbunden ist.

10. Vorrichtung nach Anspruch 9, dadurch gekennzeichnet, daß mindestens einer der weiteren Reaktoren (2) eine Verbindungsleitung (7) mit dem ersten Reaktor (1) aufweist, und/oder daß der untere Teil der Rektifikationskolonne (6) über eine Leitung (9) mit dem Kopfteil eines der weiteren Reaktoren (2) verbunden ist, und/oder daß der Abscheider (13) mit einer weiteren Rektifikationskolonne (21) verbunden ist.

## Claims

1. A process for the continuous preparation of alkyl esters of (meth)acrylic acid by reacting (meth)acrylic acid and a monohydric alkanol of 1 to 8 carbon atoms in the homogeneous, liquid, solvent-free phase at elevated temperatures and in the presence of an acidic esterification catalyst, in which the (meth)acrylic acid, the alkanol and the acid catalyst are fed continuously to a reaction zone, the alkyl (meth)acrylate formed is separated off by rectification, after a residence time, via the top of a rectification zone mounted on the reaction zone, as a component of at least one azeotropic mixture consisting of water or water and starting alkanol as further components in addition to the alkyl (meth)acrylate, the resulting distillate is separated into at least one organic phase containing the alkyl (meth)acrylate and into at least one water-containing aqueous phase, a part of the organic phase containing alkyl (meth)acrylate is recycled via the top of the rectification zone and water is recycled to the reaction zone, the alkyl (meth)acrylate is isolated from the excess organic phase containing the alkyl (meth)acrylate and a part of the reaction mixture is discharged continuously from the reaction zone, wherein the reaction zone consists of a cascade of at least two reaction regions, connected in series, and the discharge stream of one reaction region forms a feed stream of a subsequent reaction region.

2. A process as claimed in claim 1, wherein the cascade has from two to four reaction regions spatially separated from one another or wherein the ascending vapors from the reaction regions are fed to an individual rectification zone whose liquid reflux is recycled only to the first reaction region, or wherein the first reaction region is connected on the gas side neither to a downstream reaction region nor to the rectification zone.

3. A process as claimed in claim 1 or 2, wherein the temperature in the first reaction region is from 80 to 125°C and that in the last reaction region is below 135°C, or wherein the top pressure of the rectification column is from 0.1 to 1, preferably 1, atm, or wherein the total residence time of the reactants in the cascade is from 1 to 20 hours.

4. A process as claimed in any of the preceding claims, wherein the molar alkanol/(meth)acrylic acid ratio used is ≥ 1, preferably from 1 : 1 to 3 : 1, preferably from 1.1 : 1 to 1.8 : 1.

5. A process as claimed in any of the preceding claims, wherein sulfuric acid or an organic sulfonic acid, preferably methanesulfonic acid, benzenesulfonic acid, dodecylbenzenesulfonic acid or p-toluenesulfonic acid, is used as the esterification catalyst.

6. A process as claimed in any of the preceding claims, wherein the content of catalytically active acid in the first reactor region is from 0.1 to 5% by weight, based on the reaction mixture contained therein, of sulfuric acid or an equimolar amount of organic sulfonic acid, or wherein the content of catalytically active acid in the last reaction zone is from 5 to 20% by weight, based on the reaction mixture contained therein, of sulfuric acid or an equimolar amount of organic sulfonic acid, or wherein the acid to be esterified is acrylic acid, or wherein the alkanol to be esterified is a C₁-C₄-alkanol, preferably n-butanol.

7. Apparatus for carrying out a process for the continuous preparation of alkyl esters of (meth)acrylic acid as claimed in any of claims 1 to 6, having a first reactor (1) which is provided with a feed line (3) for the reactants and whose top is connected via a line (5) to the lower part of a rectification column (6) whose top is connected via a condenser to a separator (13) which has a discharge line (20) for the crude product, wherein the lower end of the first reactor (1) is connected via a line (4) to the lower end of at least one further reactor (2) whose top is connected via a line (8) to the lower part of the rectification column (6), and wherein the upper part of the separator (13) is connected to the top part of the rectification column (6) via a line (18) and the lower part of the separator (13) is connected to the top part of the rectification column (6) via a line (14/15) or to the top part of at least one of the further reactors (2) via a line (14/16).

8. Apparatus as claimed in claim 7, wherein the upper part of at least one further reactor (2) is connected via a line (7) to the upper part of the first reactor (1), or wherein the lower part of the rectification column (6) is connected via a line (9) to the top part of the first reactor (1), or wherein the separator (13) is connected to a further rectification column (21).

9. Apparatus for carrying out a process for the continuous preparation of alkyl esters of (meth)acrylic acid as claimed in any of claims 1 to 6, having a reactor (1) which is provided with a feed line (3) for the reactants and which, as a dwell container, forms the first reaction region, wherein the first reactor (1) has a liquid-side connection (4) to at least one further reactor (2) whose top is connected via a line (8) to the lower part of the rectification column (6) whose top is connected via a condenser to a separator (13) which has a discharge line (20) for the crude product, and wherein the upper part of the separator (13) is connected to the top part of the rectification column (6) via a line (18) and the lower part of the separator (13) is connected to the top part of the rectification column (6) via a line (14/15) or to the top part of at least one of the further reactors (2) via a line (14/16).

10. Apparatus as claimed in claim 9, wherein at least one of the further reactors (2) has a connecting line (7) to the first reactor (1), or wherein the lower part of the rectification column (6) is connected via a line (9) to the top part of one of the further reactors (2), or wherein the separator (13) is connected to a further rectification column (21).

## Revendications

1. Procédé de préparation continue d'esters alkyliques de l'acide (méth)acrylique, par réaction d'acide (méth)acrylique et de monoalcanols ayant de 1 à 8 atomes de carbone dans une phase homogène, liquide, exempte de solvant, à haute température et en présence d'un catalyseur acide d'estérification, dans lequel on envoie en continu dans une zone de réaction l'acide (méth)acrylique, l'alcanol et le catalyseur acide ; après un certain temps de séjour, on sépare par rectification, en tête d'une zone de rectification disposée au-dessus de la zone de réaction, l'ester alkylique ainsi formé de l'acide (méth)acrylique, en tant que constituant d'un azéotrope qui, outre l'ester alkylique de l'acide (méth)acrylique, comprend en tant qu'autres constituants de l'eau, ou de l'eau et l'alcanol de départ ; on subdivise le distillat ainsi obtenu en au moins une phase organique contenant l'ester alkylique de l'acide (méth)acrylique et au moins une phase aqueuse contenant de l'eau ; on renvoie en tête de la zone de rectification une partie de la phase organique contenant l'ester alkylique de l'acide (méth)acrylique ; et on renvoie l'eau dans la zone de réaction, on sépare l'ester alkylique de l'acide (méth)acrylique de la phase organique en excès contenant l'ester alkylique de l'acide (méth)acrylique, et on soutire en continu de la zone de réaction une partie du mélange réactionnel, caractérisé en ce que la zone de réaction est constituée d'une cascade d'au moins deux zones de réaction montées l'un derrière l'autre, le courant de sortie d'une zone de réaction formant un courant de charge d'une zone de réaction suivante.

2. Procédé selon la revendication 1, caractérisé en ce que la cascade comporte de deux à quatre zones de réaction séparées l'une de l'autre, dans l'espace et/ou en ce que les vapeurs ascendantes provenant des zones de réaction sont envoyées à une zone de rectification unique, dont le reflux liquide n'est renvoyé que dans la première zone de réaction, et/ou en ce que la première zone de réaction n'est, côté gaz, en communication ni avec une zone de réaction suivante, ni avec la zone de rectification.

3. Procédé selon la revendication 1 ou 2, caractérisé en ce que la température dans la première zone de réaction est comprise entre 80 et 125°C, et dans la dernière zone de réaction est inférieure à 135°C, et/ou que la pression en tête de la colonne de rectification est comprise entre 0,1 et 1 atmosphère et est de préférence de 1 atmosphère, et/ou que le temps total de séjour des réactifs dans la cascade est compris entre 1 et 20 heures.

4. Procédé selon l'une des revendications précédentes, caractérisé en ce que le rapport en moles entre la quantité introduite d'alcanol et celle d'acide (méth)acrylique est ≥ 1, de préférence de 1:1 à 3:1, de préférence de 1,1:1 à 1,8:1.

5. Procédé selon l'une des revendications précédentes, caractérisé en ce qu'on utilise en tant que catalyseurs d'estérification l'acide sulfurique et/ou des acides sulfoniques organiques, de préférence l'acide méthanesulfonique, l'acide benzènesulfonique, l'acide dodécylbenzènesulfonique et/ou l'acide p-toluènesulfonique.

6. Procédé selon l'une des revendications précédentes, caractérisé en ce que la teneur de la première zone de réaction en acide catalytiquement actif est, par rapport au mélange réactionnel qui y est contenu, de 0,1 à 5 % en poids d'acide sulfurique et/ou d'une quantité équimolaire d'un acide sulfonique organique, et/ou en ce que la teneur de la dernière zone de réaction en acide catalytiquement actif est, par rapport au mélange réactionnel qui y est contenu, comprise entre 5 et 20 % en poids d'acide sulfurique et/ou d'une quantité équimolaire d'un acide sulfonique organique, et/ou que l'acide à estérifier est l'acide acrylique, et/ou que l'alcanol à estérifier est un alcanol en C₁ à C₄, de préférence le n-butanol.

7. Appareil pour mettre en oeuvre le procédé de préparation continue d'esters alkyliques de l'acide (méth)acrylique selon l'une des revendications 1 à 6, comportant un premier réacteur (1), qui est pourvu d'une conduite d'amenée (3) pour les réactifs, et dont la tête communique par l'intermédiaire d'une conduite (5) avec la partie inférieure d'une colonne de rectification (6), dont la tête communique par l'intermédiaire d'un condenseur avec un séparateur (13) qui possède une conduite d'évacuation (20) pour le produit brut, caractérisé en ce que l'extrémité inférieure du premier réacteur (1) communique par l'intermédiaire d'une conduite (4) avec l'extrémité inférieure d'au moins un autre réacteur (2), dont la tête communique, par l'intermédiaire d'une conduite (8), avec la partie inférieure de la colonne de rectification (6), et en ce que la partie supérieure du séparateur (13) communique avec la partie tête de la colonne de rectification (6) par l'intermédiaire d'une conduite (18), et la partie inférieure du séparateur (13) communique avec la partie de tête de la colonne de rectification (6) par l'intermédiaire d'une conduite (14/15), et/ou, par l'intermédiaire d'une conduite (14/16), avec la partie de tête d'au moins l'un des autres réacteurs (2).

8. Appareil selon la revendication 7, caractérisé en ce que la partie supérieure d'au moins un autre réacteur (2) communique par l'intermédiaire d'une conduite (7) avec la partie supérieure du premier réacteur (1), et/ou que la partie inférieure de la colonne de rectification (6) communique par l'intermédiaire d'une conduite (9) avec la partie de tête du premier réacteur (1), et/ou que le séparateur (13) communique avec une autre colonne de rectification (21).

9. Appareil pour mettre en oeuvre le procédé de préparation continue d'esters alkyliques de l'acide (méth)acrylique selon l'une des revendications 1 à 6, comportant un réacteur (1), qui est pourvu d'une conduite d'amenée (3) pour les réactifs et qui en tant que récipient de séjour forme la première zone de réaction, caractérisé en ce que le premier réacteur (1) possède une liaison (4), côté liquide, avec au moins un autre réacteur (2), dont la tête communique par l'intermédiaire d'une conduite (8) avec la partie inférieure de la colonne de rectification (6), dont la tête communique par l'intermédiaire d'un condenseur avec un séparateur (13), qui comporte une conduite d'évacuation (20) pour le produit brut, et en ce que la partie supérieure du séparateur (13) communique avec la partie tête de la colonne de rectification (6) par l'intermédiaire d'une conduite (18), et la partie inférieure du séparateur (13) communique avec la partie de tête de la colonne de rectification (6) par l'intermédiaire d'une conduite (14/15), et/ou, par l'intermédiaire d'une conduite (14/16), avec la partie de tête d'au moins l'un des autres réacteurs (2).

10. Appareil selon la revendication 9, caractérisé en ce qu'au moins l'un des autres réacteurs (2) comporte une conduite de liaison (7) avec le premier réacteur (1), et/ou que la partie inférieure de la colonne de rectification (6) communique par l'intermédiaire d'une conduite (9) avec la partie de tête de l'un des autres réacteurs (2), et/ou que le séparateur (13) communique avec une autre colonne de rectification (21).
